(19) Europäisches Patentamt
European Patent Office
Office européen des brevets

(11) Veröffentlichungsnummer: **0 178 363**
A1

# (12) EUROPÄISCHE PATENTANMELDUNG

(21) Anmeldenummer: **84810505.2**

(22) Anmeldetag: **15.10.84**

(51) Int. Cl.[4]: **C 07 F 15/02**
//C07C101/26

(43) Veröffentlichungstag der Anmeldung:
**23.04.86 Patentblatt 86/17**

(84) Benannte Vertragsstaaten:
**CH DE GB LI NL**

(71) Anmelder: **CIBA-GEIGY AG**
**Klybeckstrasse 141**
**CH-4002 Basel(CH)**

(72) Erfinder: **Smith, Nelson**
**118 e DuRhu Drive**
**Mobile Alabama 36608(US)**

(72) Erfinder: **Stutts, Joseph W.**
**420 E. Clinton Street**
**Jackson Alabama 36545(US)**

(54) **Verfahren zur Herstellung des Eisen (III) chelats von N-(2-Hydroxyäthyl)-äthylendiamintriessigsäure.**

(57) Es wird ein Verfahren zur Herstellung des Eisen(III)chelats der N-(2-Hydroxyäthyl)-äthylendiamintriessigsäure beschrieben, das auf der Umsetzung einer wässrigen Lösung des Trinatriumsalzes der N-(2-Hydroxyäthyl)-äthylendiamintriessigsäure mit metallischem Eisen in Gegenwart von Salpetersäure und der nachfolgenden Oxidation des gebildeten Eisen(II)chelats mit einem sauerstoffhaltigem Gas beruht.

EP 0 178 363 A1

CIBA-GEIGY AG
Basel (Schweiz)

5-14624/=

## Verfahren zur Herstellung des Eisen(III)chelats von N-(2-Hydroxyäthyl)-äthylendiamintriessigsäure

Die vorliegende Erfindung betrifft ein Verfahren zur Herstellung des Eisen(III)chelats der N-(2-Hydroxyäthyl)-äthylendiamintriessigsäure.

Metallchelate sind nützliche Hilfsmittel zur Behebung von Mängeln an Spurenelementen in Böden, die für den Anbau von Zierpflanzen, Obst und Gemüse bestimmt sind. Eines der wesentlichen Spurenelemente für diese Pflanzen ist Eisen. Da Eisen in Böden und Düngemitteln weit verbreitet ist, muss nur selten zusätzliches Eisen auf den Anbauflächen ausgebracht werden. Eine Ausnahme bilden alkalische Böden, wo viele Arten von Pflanzen von Chlorose befallen werden, wenn diese Böden nicht mit Eisensalzen behandelt werden, die unter alkalischen Bedingungen löslich sind. Aus diesem Grund ist es beispielsweise erforderlich, Ananas-Kulturen mit derartigen löslichen Eisensalzen zu düngen, wenn sie auf manganhaltigen Böden gepflanzt werden.

Es sind bereits eine Reihe von organischen Verbindungen bekannt geworden, die befähigt sind mit mehrwertigen Metallen, wie z.B. Eisen, Chelate zu bilden. Zu den besten bekannten Chelatbildnern gehört N-(2-Hydroxyäthyl)-äthylendiamintriessigsäure. Sie bildet vor allem mit Eisen, Kupfer, Zink, Mangan und Calzium-Chelate. Die für den Ackerbau wichtigen Spurenelemente Eisen, Kupfer und Zink werden

bereits in Böden mit einem pH-Wert von 6 - 7 in unlösliche Hydroxyde oder in unlösliche basische Salze überführt, die von der Pflanze nicht aufgenommen werden können. N-(2-Hydroxyäthyl)-äthylendiamintriessigsäure hält diese Spurenelemente unter Chelatbildung löslich und damit für die Pflanze verfügbar. Beispielsweise bleibt Eisen in Böden mit einem pH-Wert von 10 und darüber löslich.

Als handelsübliches Eisenchelat ist insbesondere das Eisen(III)-chelat der N-(2-Hydroxyäthyl)-äthylendiamintriessigsäure zu nennen. Es kann als wässrige Lösung mit einem Gehalt von 5 % Eisen(II)ion $Fe^{3+}$ oder als kristallisiertes Salz im Handel bezogen werden. Es ist u.a. auch unter der Bezeichnung HO-EDTA Eisen(III) oder als [(N-(2-Hydroxyäthyl)-äthylendinitriolo)-triaceto]ferrat (CAS Register Nr. 17084-02-5) der Formel

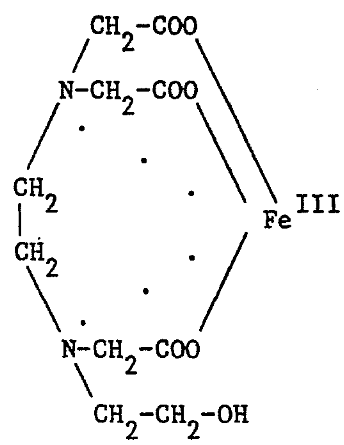

bekanntgeworden.

Diese Verbindung wurde bisher in technischem Massstab durch Umsetzung des Trinatriumsalzes der N-(2-Hydroxyäthyl)-äthylendiamintriessigsäure mit Eisen(III)sulfat hergestellt. Bei diesem bekannten Verfahren wird Natriumsulfat als Nebenprodukt gebildet, welches nach einer teuren und aufwendigen Kühlung mit Sole durch Filtration als Dekahydrat abgetrennt wird. Dabei belaufen sich die Verluste an Natriumsulfat-dekahydrat ungefähr auf 10 - 20 %. Aus der so erhaltenen Lösung des Eisen(III)chelats von N-(2-Hydroxyäthyl)-äthylendiamintriessigsäure scheidet sich bei der nachfolgenden Lagerung und/oder beim Transport weiteres Natriumsulfat ab. Das Natriumsulfat wird verworfen, da für ein derartig verunreinigtes Produkt keine Nachfrage besteht.

Das Eisen(III)chelat der N-(2-Hydroxyäthyl)-äthylendiamintriessigsäure kann auch durch Umsetzung des Trinatriumsalzes der N-(2-Hydroxyäthyl)-äthylendiamintriessigsäure mit Eisen(III)nitrat hergestellt werden. Das so hergestellte Produkt ist teurer als das mit Eisen(III)sulfat hergestellte Chelat, ist aber im Bezug auf die Abscheidung von Natriumsulfat stabiler.

Das erfindungsgemässe Verfahren beruht auf der Umsetzung des Trinatriumsalzes der N-(2-Hydroxyäthyl)-äthylendiamintriessigsäure ($HO\text{-}EDTA\bullet Na_3$) mit metallischem Eisen (Fe) in Gegenwart von Salpetersäure ($HNO_3$) und der nachfolgenden Oxidation des gebildeten Eisen(II)chelats ($HO\text{-}EDTA\bullet Fe\bullet Na$) der N-(2-Hydroxyäthyl)-äthylendiamintriessigsäure mit Sauerstoff oder einem sauerstoffhaltigem Gas zum gewünsschten Eisen(III)Chelat ($Ho\text{-}EDTA\bullet Fe$) und kann durch die folgenden Reaktionsgleichungen beschrieben werden:

a) <u>Bildung des Eisen(II)chelats</u>

$$4\ HO\text{-}EDTA\bullet Na_3+10\ HNO_3+4Fe \longrightarrow 4\ HO\text{-}EDTA\bullet Fe\bullet Na+NH_4NO_3+8NaNO_3+3H_2O$$

b) <u>Oxidation des Eisen(II)chelats</u>

$$2\ HO\text{-}EDTA\bullet Fe\bullet Na+H_2O+1/2O_2 \longrightarrow 2\ HO\text{-}EDTA\bullet Fe+NaOH$$

Das erfindungsgemässe Verfahren ist daher dadurch gekenzeichnet, dass man

a) einer wässrigen Lösung des Trinatriumsalzes der N-(2-Hydroxyäthyl)-äthylendiamintriessigsäure mindestens 2.5 Mol Salpetersäure pro Mol Trinatriumsalz zufügt,

b) der erhaltenen Mischung bei einer Temperatur unter 80°C die zur Bildung des Eisen(II)chelats der N-(2-Hydroxyäthyl)-äthylendiamintriessigsäure erforderliche Menge metallisches Eisen zusetzt,

c) der erhaltenen Lösung des Eisen(II)chelats die zur Bildung des Eisen(III)chelats notwendige Menge Trinatriumsalz der N-(2-Hydroxyäthyl)-äthylendiamintriessigsäure zusetzt,

d) das Reaktionsgemisch zur Ueberführung des Eisen(II)chelats in das Eisen(III)chelat bei einer Temperatur von 50 - 90°C mit Sauerstoff oder einem sauerstoffhaltigen Gasgemisch in Kontakt bringt und

e) den pH-Wert der erhaltenen Lösung des Eisen(III)chelats der N-(2-Hydroxyäthyl)-äthylendiamintriessigsäure auf 5,0 - 6,0 einstellt.

Das Trinatriumsalz der N-(2-Hydroxyäthyl)-äthylendiamintriessigsäure wird in der Regel als wässrige Lösung eingesetzt. Besonders geeignet ist die im Handel befindliche 40 - 50 %ige wässrige Lösung, die einen pH-Wert von etwa 10 aufweist. Die Salpetersäure wird mindestens in etwa stöchiometrischer Menge verwendet, d.h. es werden pro Mol Trinatriumsalz der N-(2-Hydroxyäthyl)-äthylendiamintriessigsäure mindestens 2,5 Mol Salpetersäure eingesetzt. In der Regel wird ein Ueberschuss von Salptersäure verwendet. Vorzugsweise verwendet man 3 -6 Mol Salpetersäure pro Mol Trinatriumsalz der N-(2-Hydroxyäthyl)-äthylendiamintriessigsäure. Bei Zugabe dieser Menge Salpetersäure resultiert ein pH-Wert von 1.0-1.4. Die Salpetersäure wird vorteilhaft in konzentrierter Form verwendet. Als besonders geeignet hat sich 50 -70 %ige Salpetersäure erwiesen. Bei der Zugabe der Salpetersäure erhöht sich die Temperatur des Reaktionsgemisches auf etwa 45°C.

Das Eisen wird im wesentlichen in stöchiometrischer Menge zugesetzt, d.h. pro Mol Trinatriumsalz der N-(2-Hydroxyäthyl)-äthylendiamintriessigsäure wird ein Grammatom Eisen verwendet. Das Eisen wird vorteilhaft in feinverteilter Form, beispielsweise in Form von Eisenpfeilspänen oder Eisenpulver zugesetzt. Als besonders vorteilhaft hat sich die Verwendung von Eisenpulver erwiesen. Das Eisen wird portionsweise mit solcher Geschwindigkeit zugegeben, dass sich das Reaktionsgemisch nicht über 80°C erwärmt. Vorzugsweise erfolgt die Zugabe des Eisen bei einer Temperatur von 50 - 80°C. Die Ueberführung des metallischen Eisens in das Eisenchelat ist eine exoterme Reaktion, die bei den vorgenannten milden Bedingungen (50 - 80°C; pH 1 - 2) hauptsächlich unter Bildung von Ammoniumnitrat ($NH_4NO_3$) verläuft. Bei Temperaturen über 80°C können auch Wasserstoff und Stickoxide als Nebenprodukte gebildet werden. Auch bei einem pH-Wert von unter 1 kann die Reaktion heftiger verlaufen, was zu einer teilweisen Oxidation des organischen Materials führt.

Die Zugabe des Eisen erfolgt vorzugsweise in Zeitintervallen von 10 Minuten, damit die Wärme durch Kühlung mit Luft oder, falls notwendig, durch Kühlung mit einem mit Wasser beschickten Kühlmantel abgeführt werden kann. In der Regel kann das gesamte Eisen während 2 Stunden zugegeben werden. Nach Zugabe des Eisens beträgt der pH-Wert des Reaktionsgemisches etwa 1,8.

Nach Zugabe der gesamten Menge des Eisens liegt eine wässrige Lösung des Eisen(II)chelats der N-(2-Hydroxyäthyl)-äthylendiamintriessigsäure vor, die zwei Drittel der für die Bildung des Eisen(III)-chelats erforderliche Menge an N-(2-Hydroxyäthyl)-äthylendiamintriessigsäure enthält. Die Oxidation von nichtcheliertem Eisen(II)-ionen zu Eisen(III)ionen ist in wässriger Lösung thermodynamisch ungünstig. Da das Eisen(III)chelat einen stärkeren Komplex darstellt als das entsprechende Eisen(II)chelat, bewirkt die Gegenwart von N-(2-Hydroxyäthyl)-äthylendiamintriessigsäure eine Verschiebung des Gleichgewichts zu Gunsten der Eisen(III)oxidationsstufe. Es ist daher vorteilhaft, die zur Bildung des Eisen(III)chelats erforderliche Menge N-(2-Hydroxyäthyl)-äthylendiamintriessigsäure vor dem

Beginn der Oxidation zuzusetzen. Die zur Bildung des Eisen(III)-chelats aus dem Eisen(II)chelat erforderliche Menge an N-(2-Hydroxyäthyl)-äthylendiamintriessigsäure wird dem Reaktionsgemisch vorteilhaft in Form des Trinatriumsalzes zugesetzt. Insgesamt werden 95 - 100 % der stöchiometrischen Menge an Trinatriumsalz der N-(2-Hydroxyäthyl)-äthylendiamintriessigsäure zugesetzt. Anschliessend wird das Reaktionsgemisch auf 90°C aufgeheizt und unter Rühren Luft oder ein anderes sauerstoffhaliges Gas durchgeleitet.

Während der Oxidation verändert sich die Farbe des Reaktionsgemisches allmählich von dunkelgrün nach dunkelrot. Das fortschreitende Oxidation kann leicht durch Titrieren von Proben des Reaktionsgemisches mit 0,1 N Cer(IV)sulfat unter Verwendung von Ferrion als Indikator verfolgt werden. Dabei wird 1 ml der Probe in 75 ml 7N Schwefelsäure gegeben und titriert. Wenn die Konzentration an Eisen(II)ionen unter 0,1 Gewichtsprozent abfällt, ist die Farbe der schwefelsauren Lösung fast farblos, während die Lösung bei einem höheren Gehalt an Eisen(II)ionen eine blassgrüne Färbung zeigt.

Für die Oxidation wird vorzugsweise Luft als Oxidationsmittel verwendet. Es kann jedoch auch jedes andere sauerstoffhaltige Gas oder reiner Sauerstoff verwendet werden.

Die Zeit, die für die Oxidation benötigt wird, hängt wesentlich von der Rührgeschwindigkeit und der Geschwindigkeit der Zufuhr von Sauerstoff ab. Nach Beendigung der Oxidation liegt der pH-Wert des Reaktionsgemisches im Bereich von 5,2 - 5,8.

Das im Handel befindliche Produkt enthält gemäss Spezifikation einen Ueberschuss von N-(2-Hydroxyäthyl)-äthylendiamintriessigsäure von 0,1 - 1,0 Gewichtsprozent. Der pH-Wert des Produkts kann entweder durch Zugabe von Trinatriumsalz der N-(2-Hydroxyäthyl)-äthylendiamintriessigsäure, wässrigem Alkalimetallhydroxid oder von Salpetersäure leicht auf den durch die Spezifikation vorgesehenen Wert eingestellt werden.

Schliesslich wird der Eisengehalt des Produktes durch Zugabe von Wasser auf 5,0 - 5,5 Gewichtsprozent eingestellt und nichtumgesetztes Eisen beziehungsweise Eisenoxid abfiltriert.

Die Materialkosten für das erfindungsgemässe Verfahren liegen etwa ein Drittel billiger als bei dem bekannten, auf der Umsetzung des Trinatriumsalzes der N-(2-Hydroxyäthyl)-äthylendiamintriessigsäure mit 50 %iger Eisen(III)sulfatlösung basierenden Verfahren. Ferner ist der direkte Zeit- und Arbeitsaufwand ebenfalls viel niedriger und ausserdem entfällt die Solekühlung zur Abscheidung von Verunreinigungen durch Kristallisation.

Mit der Durchführung des erfindungsgemässen Verfahrens ist nur eine äusserst geringe Belastung der Umwelt durch Abfallprodukte verbunden. So fallen pro 1000 kg Produkt lediglich 8 kg Rückstand, in erster Linie Eisenoxide, an. Dieser Rückstand ist unschädlich und kann leicht durch Vergraben beseitigt werden. Ferner werden pro 1000 kg Produkt etwa 9 kg Stickoxide gebildet. Diese bestehen überwiegend aus Stickstoffdioxid, da Stickstoffmonoxid in Gegenwart von Sauerstoff leicht in Stickstoffdioxid umgewandelt wird. Die Hauptmenge des Stickstoffdioxids wird in den letzten 30 Minuten der Luftoxidation beobachtet, wenn die Hauptmenge des Eisens bereits oxidiert ist und die Konzentration von Eisen(II)chelat weniger als 1 Gewichtsprozent beträgt. Dies zeigt den Beginn der Oxidation von organischer Substanz an. Das Stickstoffdioxid kann aus dem Abgas in effizienter Weise durch Waschen mit Wasser und Ueberführung in Salpetersäure entfernt werden.

Untersuchungen über die Korrosion der Reaktionsgefässe während der höchsten Belastungen, d.h. am Beginn des Verfahrens, wenn der pH-Wert am tiefsten ist und bei der Oxidation bei 90°C, zeigen dass das gesamte Verfahren in Gefässen aus rostfreiem Stahl durchgeführt werden kann.

Das erfindungsgemässe Verfahren wird durch die nachfolgenden Beispiele näher erläutert:

Beispiel 1: In einem Glasreaktor werden 470 g einer 44,8 %igen wässrigen Lösung des Trinatriumsalzes der N-(2-Hydroxyäthyl)-äthylendiamintriessigsäure (210,6 g (0,61 Mol) HO-EDTA•$Na_3$; CHEL DM ®) vorgelegt und 252 g 70 %ige Salpetersäure (176,4 g (2,8 Mol) $HNO_3$) aus einem Tropftrichter zugegeben. Durch die einsetzende exotherme Reaktion steigt die Temperatur auf 45°C und der pH-Wert fällt von anfangs 10 auf etwa 1,2. Dann werden bei 50°C 54,7 g Eisenpulver (90 %) in das Reaktionsgemisch eingetragen. Die Zugabe des Eisens erfolgt in Anteilen mit einem zeitlichen Abstand von jeweils 10 Minuten. Bei dieser Art der Zugabe kann die entwickelte Wärme durch Kühlung des Reaktors mit Luft abgeführt werden. Nach beendigter Zugabe des Eisens resultiert eine dunkelgrüne wässrige Lösung des Eisen(II)chelats der N-(2-Hydroxyäthyl)-äthylendiamintriessigsäure. Dieser Lösung werden weitere 174 g 44,8 %ige wässrige des Trinatriumsalzes der N-(2-Hydroxyäthyl)-äthylendiamintriessigsäure zugefügt. Anschliessend wird bei 90°C unter Rühren Luft durch das Reaktionsgemisch geleitet. Während des Einleitens von Luft ändert sich die Farbe der Lösung allmählich von dunkelgrün nach dunkelrot.

Das Fortschreiten der Oxidation wird durch laufende Bestimmung des Eisen(II)gehalts durch Filtration von Proben des Reaktionsgemisches mit Cer(IV)sulfat bestimmt. Nach 2,5 Stunden ist die Oxidation beendet und es liegt eine dunkelrote Lösung des Eisen(III)chelats vor. Nun wird soviel Trinatriumsalz der N-(2-Hydroxyäthyl)-äthylendiamintriessigsäure zugesetzt, dass sich bezogen auf Eisen ein Ueberschuss von N-(2-Hydroxyäthyl)-äthylendiamintriessigsäure von 0,1 - 1,0 % ergibt, der pH-Wert der Lösung wird dann durch Zugabe von Salpetersäure oder Trinatriumsalz der N-(2-Hydroxyäthyl)-äthylendiamintriessigsäure auf 5,5 - 5,8 gestellt und der Eisengehalt der Lösung wird durch Zugabe von Wasser auf 5,0 - 5,5 Gewichtsprozent gebracht.

Die Ausbeute an Eisen(III)chelat beträgt 96 % bezogen auf eingesetztes Trinatriumsalz der N-(2-Hydroxyäthyl)-äthylendiamintriessigsäure.

Beispiel 2: In einem 7,5 $m^3$ Reaktor aus rostfreiem Stahl werden 3218 l 43 %ige Lösung des Trinatriumsalzes der N-(2-Hydroxyäthyl)-äthylendiamintriessigsäure vorgelegt. Dann wird Kühlwasser durch den Kühlmantel des Reaktors geleitet und unter Rühren 1700 l 67 %iges Salpetersäure zugegeben. Nach der Zugabe der Salpetersäure beträgt der pH-Wert der Mischung 1,0 - 1,4.

In die erhaltene Mischung werden insgesamt 522 kg Eisenpulver (90 %) in Anteilen von etwa 50 kg in Zeitintervallen von 10 Minuten eingetragen. Bei der Zugabe des Eisen steigt die Temperatur bei voll eingeschalteter Kühlung allmählich auf 75 - 80°C. Nach vollständiger Zugabe des Eisens wird das Reaktionsgemisch noch 20 - 30 Minuten ohne Kühlung gerührt um eine vollständige Umsetzung des Eisens zu gewährleisten.

Danach werden weitere 1287 l 43 %ige Lösung des Trinatriumsalzes der N-(2-Hydroxyäthyl)-äthylendiamintriessigsäure zugegeben, wobei der pH-Wert der Mischung auf 3,5 - 4,5 steigt. Anschliessend werden unter vermindertem Druck etwa 550 - 600 l Wasser abdestilliert. Dann wird bei 90°C ohne weitere Kühlung 7 Stunden Luft durch das Reaktionsgemisch geleitet, wobei die Temperatur allmählich auf 60 - 70°C absinkt. Nach beendigter Oxidation wird soviel Trinatriumsalz der N-(2-Hydroxyäthyl)-äthylendiamintriessigsäure zugesetzt, dass sich bezogen auf Eisen ein Ueberschuss an N-(2-Hydroxyäthyl)-äthylendiamintriessigsäure von 0,1 - 1,0 % ergibt. Der pH-Wert der Lösung wird durch Zugabe von Salpetersäure oder Natronlauge auf 5,2 - 5,8 gestellt. Dann wird filtriert und der Eisengehalt durch Zugabe von Wasser auf 5,0 - 5,5 Gewichtsprozent gebracht. Die Ausbeute beträgt 98 % Eisen(III)chelat bezogen auf eingesetztes Trinatriumsalz der N-(2-Hydroxyäthyl)-äthylendiamintriessigsäure.

Patentansprüche

1. Verfahren zur Herstellung des Eisen(III)chelats der N-(2-Hydroxyäthyl)-äthylendiamintriessigsäure, dadurch gekennzeichnet, dass man

a) einer wässrige Lösung des Trinatriumsalzes der N-(2-Hydroxyäthyl)-äthylendiamintriessigsäure mindedestens 2,5 Mol Salpetersäure pro Mol Trinatriumsalz zufügt,

b) der erhaltenen Mischung bei einer Temperatur unter 80°C die zur Bildung des Eisen(II)chelats der N-(2-Hydroxyäthyl)-äthylendiamintriessigsäure erforderliche Menge metallisches Eisen zusetzt,

c) der erhaltenen Lösung des Eisen(II)chelats die zur Bildung des Eisen(III)chelats notwendige Menge Trinatriumsalz der N-(2-Hydroxyäthyl)-äthylendiamintriessigsäure zusetzt,

d) das Reaktionsgemisch zur Ueberführung des Eisen(II)chelats in das Eisen(III)chelat bei einer Temperatur von 50 - 90°C mit Sauerstoff oder einem sauerstoffhaltigen Gasgemisch in Kontakt bringt und

e) den pH-Wert der erhaltenen Lösung des Eisen(III)chelats der N-(2-Hydroxyäthyl)-äthylendiamintriessigsäure auf 5,0 - 6,0 einstellt.

2. Verfahren nach Anspruch 1, dadurch gekennzeichnet, dass man eine 40 - 50 %ige wässrige Lösung des Trinatriumsalzes der N-(2-Hydroxyäthyl)-äthylendiamintriessigsäure verwendet.

3. Verfahren nach Anspruch 1, dadurch gekennzeichnet, dass man 3 - 6 Mol Salpetersäure pro Mol Trinatriumsalz der N-(2-Hydroxyäthyl)-äthylendiamintriessigsäure einsetzt.

4. Verfahren nach Ansprüchen 1 und 3, dadurch gekennzeichnet, dass man 50 - 70 %ige Salpetersäure verwendet.

5. Verfahren nach Anspruch 1, dadurch gekennzeichnet, dass man das Eisen in Form von Eisenpulver verwendet.

6. Verfahren nach Anspruch 1, dadurch gekennzeichnet, dass man die Zugabe des Eisens bei 50 - 80°C vornimmt.

7. Verfahren nach Anspruch 1, dadurch gekennzeichnet, dass man die Oxidation des Eisen(II)chelats zum Eisen(III)chelat mit Luft durchführt.

FO 7.5 GOT/bg*

Europäisches Patentamt

# EUROPÄISCHER RECHERCHENBERICHT

0178363
Nummer der Anmeldung

EP 84 81 0505

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| | | | C 07 F 15/02 // C 07 C 101/26 |
| A | DE-A-2 606 634 (AGFA-GEVAERT) * Ansprüche 1,4 * --- | 1 | |
| A | DE-A-2 334 034 (FUJI PHOTO FILM) * Anspruch 10, Beispiele 1,3,4 * --- | 1 | |
| A | US-A-3 115 511 (HAMPSHIRE CHEMICAL) * Anspruch 4 * ----- | 1 | |
| | | | RECHERCHIERTE SACHGEBIETE (Int. Cl.4) |
| | | | C 07 F 15/02<br>C 07 C 101/26 |

Der vorliegende Recherchenbericht wurde für alle Patentansprüche erstellt.

| Recherchenort | Abschlußdatum der Recherche | Prüfer |
|---|---|---|
| BERLIN | 05-06-1985 | KAPTEYN H G |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82